# EUROPEAN PATENT APPLICATION

(11) **EP 1 260 282 A2**
(43) Date of publication of application: **27.11.2002**
(21) Application number: 02447089.0
(22) Date of filing: 16.05.2002
(51) Int. Cl.: B09C 1/10, C02F 3/32

(54) **Method for improving phytoremediation treatment of a contaminated medium**

(30) Priority: 16.05.2001 US 291344 P
(71) Applicant: "VLAAMSE INSTELLING VOOR TECHNOLOGISCH ONDERZOEK", afgekort "V.I.T.O.", 2400 Mol (BE)
(72) Inventor: van der Lelie, Danièl, 2440 Geel (BE); d'Haene, Siegfried, 9031 Drongen (BE); Niall Dowling, David, Carlow (IE); Karlson, Ulrich, 4000 Roskilde (DK); Moore, Edward R. B., 38124 Braunschweig (DE); Taghavi, Safiyh, 4000 Liege (BE); Trapp, Stefan A. J., 49716 Meppen (BE); Vangronsveld, Jaco, 3590 Diepenbeek (BE)
(74) Representative: Vandersteen, Pieter

(57) **Abstract**

A method for the phytoremediation treatment of a contaminated medium with at least one element selected from the group consisting of (preferably water soluble and volatile) organic pollutants, heavy metals, radionuclides or a mixture thereof, comprising the step of cultivating upon said contaminated medium a plant associated with an endophytic microorganism able to improve the phytoremediation of said plant, to reduce phytotoxicity of chemicals, and the step of recovering the elements present in said plant.

## Description

### Field of the invention

The present invention is in the field of biotechnology and is related to the use of endophytic microorganisms, especially bacteria to improve phytoremediation of a contaminated medium, especially soils contaminated by heavy metals, radionuclides and/or organic pollutants.

### Background of the invention and state of the art

The soil pollution by toxic organic compounds is an important environmental problem- Phytoremediation may offer a possible solution or reduction of the problem. Phytoremediation is the process of using plants for *in situ* remediation of soils or groundwater contaminated with different pollutants via extraction, degradation and/or stabilization of contaminants. Phytoremediation of organic xenobiotics is based on combined action between plants and their associated microorganisms. Degradation of organic contaminants can occur in the plant rhizosphere and *in planta.*

The use of biological techniques can strongly reduce the costs of remediating sites contaminated with organic xenobiotics. For large contaminated sites, bioremediation is the only alternative economically and socially acceptable. Especially phytoremediation, one of the soft bioremediation techniques, is becoming an acceptable alternative for the treatment of contaminated sites and wastewater. Phytoremediation of organic contaminants is based on the combined action between plants and their associated microorganisms, such as mycorrhizal fungi and bacteria.

Degradation of organic xenobiotics can occur in the plant rhizosphere and *in planta.* However, certain organic pollutants may not be degraded, but may be accumulated in the plant or be volatilised through the plant leaves.

In addition, water soluble and volatile organic pollutants might be partially degraded by plants and subsequently, accumulation of toxic metabolites can occur.

Soil contaminants, especially organic xenobiotics with a log K_{ow} between 0.5 and 3.5 and weak electrolytes (weak acids and bases or amphoteres such as herbicides) are readily taken up by plants (Trapp *et al.,* 1994; Trapp 2000). Recent unpublished evidence suggests that numerous compounds (see also Table 1) enter the xylem faster than the soil microflora can degrade them, even if the rhizosphere is enriched with degrader bacteria.

**Table 1:**

| Non-exhaustive list of pollutants that provide potential problems in phytoremediation due to uptake followed by insufficient plant metabolism. | | |
|---|---|---|
| **Compound** | **Fate in plant (toxic, build up, or volatile)** | **Reference** |
| Phenols | Toxic | Pfleeger *et al.*, 1991 |
| Chloro-phenols | Toxic | Pfleeger *et al.*, 1991 |
| TNT | Toxic, degraded to amino-dinitrotoluene | Thompson *et al.*, 1998 |
| Amino-dinitrotoluene | Rather persistent, toxic | Thompson *et al.*, 1998 |
| MTBE | Volatile | Trapp *et al.,* 1994 |
| BTEX | Volatile | Trapp *et al.,* 1994 |
| TCE | Volatile, Build-up of trichloroacetate | Trapp *et al.,* 1994 |
| PER | Volatile | Trapp *et al.,* 1994 |

Although some pollutants are metabolized by plants, numerous pollutants - or their metabolites - are toxic to plants. This can seriously limit the applicability of phytoremediation (because plants do not grow correctly or may die in toxic soils) . Alternatively, in the case of volatile pollutants, plants release these compounds, or their metabolites, through the stomata, which questions the merits of an efficient phytoremediation by said plant.

Although offering some interesting benefits compared to the traditional remediation techniques, phytoremediation of contaminated medium by phytoextraction of heavy metals and radionuclides still has its limitations. A suitable plant used in extraction of heavy metals should possess several characteristics, which are rarely found within one plant species. For this reason, different strategies are currently being investigated in order to improve crops for phytoextraction processes (Cunningham & Berti, 1993; Cunningham & Ow, 1996; Burd et al., 1998; Arazi et al., 1999; Brewer et al., 1999).

Endophytic microorganisms, especially bacteria are ubiquitous in most plant species, residing latently or actively colonising plant tissues. Historically, endophytic microorganisms, especially bacteria have been thought to be weakly virulent plant pathogens, but have recently been discovered to have several beneficial effects on host plants, such as plant growth promotion and increased resistance against plant pathogens and parasites.

Endophytic bacteria have been isolated from different parts of the plants, including roots, stems and leaves. Endophytic colonisation of the vascular system (phloem, xylem) has been reported, their numbers being quite significant (10⁺³-10⁺⁶ cfu/ml). Especially in trees, such as poplar or willow that are currently used to develop phytoremediation strategies for organics, the time period between uptake of organics by the roots and their arrival in the leaves takes several hours to days (Mc Crady *et al.,* 1987; Trapp *et al.,* 2001), as the compounds travel through the vascular system.

### Aims of the present invention

The present invention aims to provide a new method and plant for improving phytoremediation, especially for water soluble and volatile organic pollutants degradation by plant and to improve treatment of toxic pollutants or their metabolites by the plant without being toxic for said plant.

Another aim of the invention is to reduce the possible volatilisation through the plant's leaves of said pollutants and their possible metabolites.

A further aim of the present invention is to improve the phytoremediation of heavy metal and radionuclides, especially improving heavy metals radionuclides uptake and translocation by plants and improving the phytoextraction of heavy metals and radionuclides of a contaminated medium, especially of a contaminated soil.

### Summary of the invention

The present invention is related to a method for the phytoremediation treatment of a medium (such as a soil or an aqueous medium), contaminated with at least one element selected from the group consisting of (preferably, water soluble or volatile) organic pollutants, heavy metals or radionuclides, said method comprising the step of cultivating in or upon said medium, a plant associated with an endophytic microorganism able to improve phytoremediation by said plant and the step of recovering the element or the degraded metabolites of said element inside the plant.

According to the invention the endophytic microorganism present in said plant is an endophytic bacteria (or an endophytic fungi).

Endophytic microorganisms, especially endophytic bacteria are defined as those microorganisms that are able to enter plant tissues and to establish themselves inter- and intra-cellularly (Di Fiori & Del Gallo, 1995). They have the ability to establish an active relationship with their hosts and can be defined as colonists (Kloepper & Beauchamp, 1992; Kloepper et al., 1992).

Said endophytic microorganism could be a isolated and purified natural microorganism or a genetically modified microorganism.

Preferably, said endophytic microorganisms are present in the vascular system (phloem, xylem) or the root system of the plant.

According to a preferred embodiment of the present invention, the endophytic microorganism is selected but not limited to the group consisting of the genera Pseudomonas, Azotobacter, Azomonas, Acinetobacter, Xanthomonas, Stenotrophomonas, Comamonas, Burkholderia, Ralstonia, Alcaligenes, Derxia, Xylella, Delftia, Rhizobium, Bradyrhizobium, Rhizomonas, Sphingomonas, Azospirillum, Blastomonas, Porphyrobacter, Zymomonas, Brevundimonas, Phenylbacterium, Agrobacterium, Chelatobacter, Sinorhizobium, Allorhizobium, Phyllobacterium, Aminobacter, Mesorhizobium, Ochrobactrum, Beijerinckia, Azorhizobium, Devosia, Nevskia, Afipia, Blastobacter, Chromobacterium, Herbaspirillum, Acidovorax, Brachymonas, Variovorax, Thauera, Zoogloea, Azoarcus, Spirillum, Rhodanobacter, Halomonas, Alcanivorax, Zymobacter, Agromonas, Chryseomonas, Flavimonas, Phenylbacterium, Rhizobacter, Moraxella, Psychrobacter, Alteromonas, Pseudoalteromonas, Shewanella, Vibrio, Photobacterium, Aeromonas, Enterobacter, Pantoea, Brenneria, Pectobacterium, Bacillus, Actinomyces, Corynebacterium, Frankia, Nocardia, Rhodococcus, Streptomyces, Flavobacterium, Flexibacter, or the group of the pink-pigmented facultatively methylotrophic bacteria.

According to a preferred embodiment of the present invention the water soluble or volatile organic pollutant is an agrochemical such as an herbicide, or a pollutant such as toluene, benzene, phenols, chlorophenols, TNT, amino-dinitrotoluene, MTBE, BTEX, chlorinated ethenes, organotin compounds, PCBs, PBBs, brominated flame retardants, fluorinated alkylsulfonates, in particular perfluoro-octanyl sulfonate (PFOS).

According to another embodiment of the present invention, the heavy metals or radionuclides are metals selected from the group consisting of zinc, cadmium, cobalt, nickel, copper, lead, mercury, thallium, barium, boric, selenium, chrome, cesium, strontium, uranium, plutonium, lanthanides or their salts.

Another aspect of the present invention is related to a plant comprising in its vascular system (phloem, xylem) a genetically modified, or a naturally occurring isolated and purified, endophytic microorganism that is able to express proteins that allow an efficient degradation or a phytoaccumulation of at least one of the elements above-mentioned (organic pollutants, heavy metals, radionuclides, or a mixture thereof).

However, by introducing endophytic organisms that express degradation genes for specific organic xenobiotics, these compounds might be efficiently degraded, resulting in no or strongly reduced build-up of these compounds or their toxic degradation intermediates in the plants or in reduced phytovolatilization.

Introduction and heterologous expression of known heavy metal resistance genes in endophytic microorganisms, especially endophytic bacteria resulting unexpectedly in an effect on the uptake capacities of heavy metals by their host plant. Salt *et al.* (1999) have shown that Cd tolerant rhizobacteria are able to promote Cd precipitation processes near the root surface of Indian mustards plants and consequently decreased the toxic effects of the metal cation for the roots. Previous studies have shown that several mechanisms can be responsible for bacterial heavy metal resistance e.g. blocking the entry of toxic ions in the cells, intracellular sequestration of the metals by metal binding proteins, enzymatic conversion of the metal to a less toxic form and energy driven efflux systems for cations and anions encoded by resistance genes, such as the *czc, cnr, ncc, cad,* and *ars* operons (Mergeay, 1997; Taghavi *et al.,* 1997).

Bio-precipitation and sequestration processes also seem to take place when bacteria are equipped with efflux mechanisms. This phenomenon was observed in cultures of *Ralstonia metallidurans* CH34 (previous *Alcaligenes eutrophus* CH34) when grown in the presence of high concentrations of Cd or Zn and attributed to the action of the *czc* resistance operon on the pMOL30 plasmid (Diels *et al.,* 1995). Such bio-precipitation and sequestration characteristics could offer interesting benefits for the bacteria, and in the case of endophytic bacteria the speciation of the heavy metals might be altered in the host plant from a free to a less available form and lead to a reduced toxicity of the heavy metals on plant metabolism.

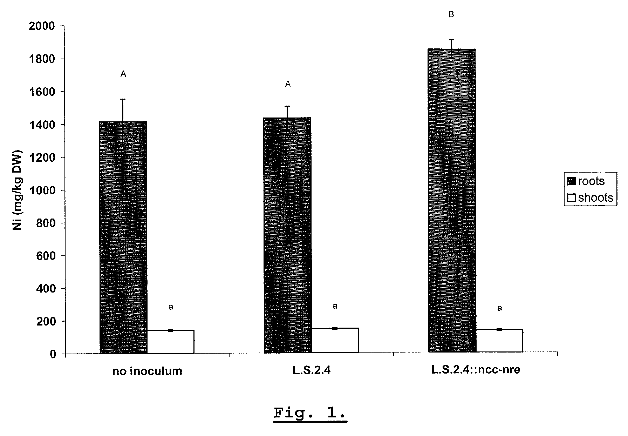

The present invention will be described in more details in the following non-limiting examples in reference to the enclosed figure 1.

### Short description of the drawing

Figure 1 represents an Ni concentration (mg/kg dry weight) in roots and shoots of *Lupinus luteus L.* plants for different inocula and following 0.25mM NiCl₂ treatment (L.S.2.4 was inoculated as the wild type strain and L.S.2.4::*ncc-nre* as its Ni resistant derivative. Data are mean values of 3 replicate samples ± S.D. Different letters indicate values that are statistically significant (P< 0.05)).

Figure 2 shows the influence of different concentration of toluene on growth of Lupinus luteus

### Examples

### Example 1: heavy metal sequestration by natural and genetically modified endophytic bacteria

*Pseudomonas* sp. VM422 was isolated as an endophytic strain from surface sterilised *Brassica napus.* This strain was selected for its zinc resistance phenotype: VM422 had a MIC value for zinc of 20 mM on Tris minimal medium (Mergeay et al., 1985). This strain was tested for its zinc complexing capacity by growing it for 66 hours in liquid medium in the presence of 60000 µg/l ZnCl₂. After the incubation period, approximately 800 µg/l Zn remained in the solution: the majority of the Zn was biosequestrated around the VM422 cells. For comparison, a similar experiment with *Ralstonia metallidurans* CH34, a well-known heavy metal sequestration bacterium (Diels et al., 1995), resulted in a decrease of Zn to 2730 µg/l in the remaining solution. This experiment demonstrates the feasibility to use natural, heavy metal resistant endophytic bacteria for heavy metal sequestration from solution.

Burkholderia cepacia L.S.2.4::*ncc-nre*, in which the miniTn*5-ncc-nre* transposon had been introduced (Taghavi et al, 2001) was examined for its efficiency for nickel sequestration. Strain L.S.2.4 was grown for 7 days in liquid medium in the presence of 25 mg/l NiCl2. After the incubation period, approximately 15 mg/l Ni remained in the solution, indicating that 40% of the Ni was sequestrated around the *B. cepacia L.S.2.4.:ncc-nre* cells. This experiment demonstrates the feasibility to use genetically modified, heavy metal resistant endophytic bacteria for heavy metal sequestration from solution.

### Example 2: construction of recombinant endophytic strains equipped with degradation pathways for specific organic xenobiotics

For construction of strains of endophytic bacteria with improved degradation capacity of organic xenobiotics (benzene, toluene, phenols and TCE) natural gene transfer was used. Natural gene transfer is based on bi or tri parental conjugation or exogenous plasmid isolation.

As a model endophytic strain to be equipped with degradation pathways was used a nickel-kanamycin marked derivative of Burkolderia cepacia L.S.2.4 named strain BU 0072, which was constructed at VITO (Taghavi, S. et all,2001). Burkolderia cepacia L.S.2.4 has yellow lupine (Lupinus luteus L.) as host.

As a donor strain for degradation pathway Burkolderia cepacia G4 (TOM, conjugative plasmid, tol⁺ ) was used.

Donor strain and receptor strain were grown overnight in LB medium, washed in 10⁻² MgSO₄ and aliquots of 100µl were added to a sterile filter (0.45µl) and incubated overnight at 30C° on solid LB medium. The filter containing donor, receptor and transconjugants were washed in a 10⁻² MgSO₄ solution in order to release the bacteria. Transconjugant was selected by means of their acquired Ni+Km+Tol resistance on minimal Tris buffered medium supplemented with the appropriate concentration of selective marker i.e. 1mM NiCl₂, 100µg/ml Km and toluene as carbon source.

After three weeks the mating between BU 0072 and BU G4 resulted in transconjugants that were Km^{R} and tol⁺, with a transfer efficiency of 7.1x10⁻³.

The presence of nre which confers the Ni resistance in the transconjugants of BU 0072 x G4 was confirmed with PCR using the following specific primers:
Sense: 5′-ggAgAgCgCCgTgACCCAggCgAAgAAggCgCTggTgCATgACCATATCgACC-3′
Antisense:

5-
CACgATCACCATggCgCTggCTgCTgCCgCggCAAGATTCAgCgCCAgCAgTCCCTTC-
3′

The strain BU0072 was used as a positive control.

Amplification was carried out as follows: a preliminary denaturation step was done at 95°C for 10 min, followed by 35 cycles of 2 min at 95°C, 1 min at 55°C, 2 min at 72°C and 8 min at 72°C. PCR product of 930 bp was checked by electrophoresis in 1,2% agarose gels. All the transconjugants showed the nre specific fragment.

The presence of pTOM was confirmed by plasmid isolation of the transconjunants and of Burkolderia cepacia G4 as positive control, while strain BU0072 was used as a negative control. A large fragment corresponding with the plasmid was present in the transconjugants and in G4, but was lacking in BU0072. One of the representative transconjugants was chosen and named VM1330.

Strain VM1330 is used to reinoculate lupine plants to prove the concept of the project.

### Example 3: development and comparison techniques for efficient reinoculation of endophytic strains in their host plants.

After having marked and equipped endophytic bacteria with degradation pathways an efficient recolonization of host plant is an important prerequisite to evaluate their contribution inside of the plant to degrade the pollutants as they are being transported trough the plant and consequently reduce phytotoxicity and volatilization of the pollutants.

### Preparation of bacterial inoculum:

A VM 1330 strain was grown in 284 tris buffered, salted, minimal liquid medium with addition of 0.2% gluconate at 22°C on rotary shaker for a period of 7 days. Next, inoculum was centrifuged at 6000rpm during 15 minutes, washed twice in MgSO₄ ⁻². Inoculum was diluted and plated on 284 medium with addition of 1mM Ni, 50 mg/l kanamicyne and toluene in order to test the purity of the solution and the presence of Ni, Km and toluene resistance characteristics.

### Seeds surface sterilization:

Seeds of Lupinus luteus were surface sterilized in solution containing 1% active chloride and 1 droplet Tween per 100ml solution. After sterilization seeds were rinsed 3 times in sterile water and dried on sterile filter paper. In order to test sterilization efficiency seeds were incubated on 869 medium during 3 days at 30°C.

### Inoculation and plant growth conditions:

Surface sterile seeds of Lupinus luteus were planted in sterile plastic jars (800ml), completely filled with sterile perlite and saturated with ½ concentrated sterile Hoagland's solution. Five seeds were planted in each jar.

Perlite was chosen as plant growth substrate because it can be sterilized easily and provides the roots with moisture, nutrients and a good aeration due to the large surface area and the physical shape of each particle.

The bacterial inoculum was added in each jar at concentration of 10⁸ colony forming units (CFU) per milliliter Hoagland's solution. Inoculum was added in MgSO₄ ⁻², whereas for the non-inoculated plants the same amount of MgSO₄ ⁻² was added.

The jars were covered with sterile tinfoil in order to allow a good bacterial colonization and prevent contamination and dispersion of the inoculated bacteria through the air. After germination of the seeds, holes were made in the tinfoil and plans were grown through the holes.

Plants were grown for 21 days in climate chamber with constant temperature 22°C, relative humidity 65% and 12 hour light and dark cycle.

### Recovery of bacteria:

After 21 days plants were harvested. Roots and shoots were separated. Fresh root and shoot material was vigorously washed in distilled water, sterilized in solution containing 1% active chloride supplemented with 1 droplet Tween per 100ml solution, rinsed 3 times in sterile distilled water. After sterilization roots and shoots were macerated using mixer in 10ml sterile MgSO₄ ⁻². 100µl of supernatant was immediately plated on three different media: 284 + 0.2% gluconate; 284+1mM Ni + 100 Km+ 0.2% gluconat and 284 + 1mM Ni +50 Km + toluene.

From the perlite growth substrate 2.5g was shaken for 30 minutes in 10ml MgSO₄ ⁻² and plated on the same media as mentioned before. Incubation for 7 days at 30°C proceeded the bacterial counting.

Results of the counting are presented in table 2.1

Those bacteria were purified on the same media and it was shown that bacteria from reinoculated plants could grow on media with addition of 1mM Ni, Kanamicyne and toluene. None bacteria from control plant have that capability.

Presence of Ni and Km resistance and presence of Tom plasmide in bacteria isolated from reinoculated plants of Lupinus luteus is confirmed by means of BOX PCR (Vito).

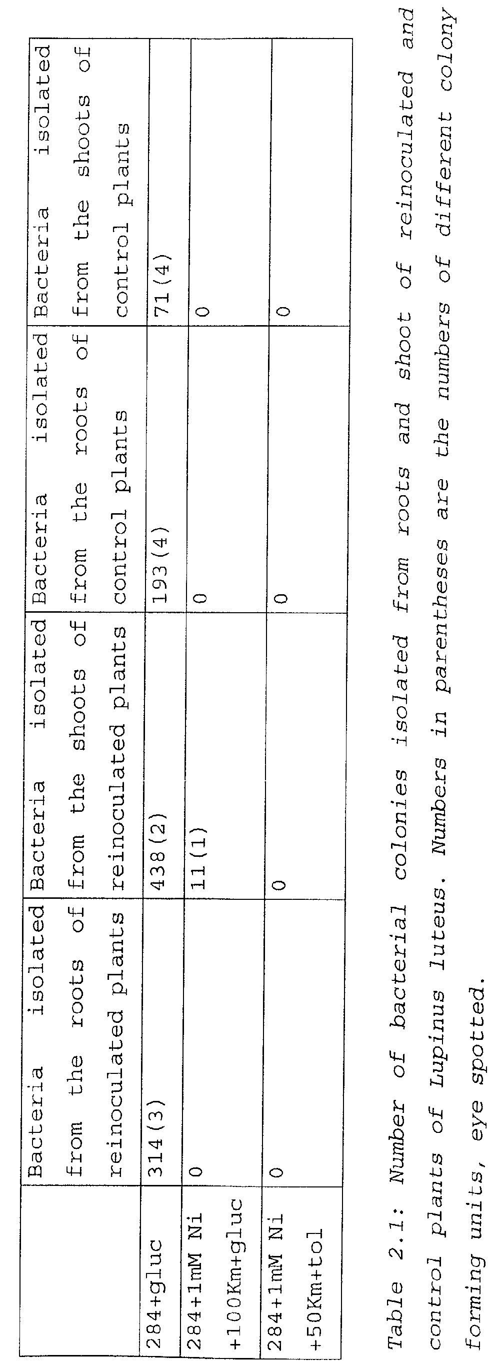

### Example 4: improved phytoaccumulation of heavy metals

In order to exploit the use of endophytic bacteria to improve the phytoextraction of heavy metals, *Burkholderia cepacia* was selected as endophytic strain. Some *B. cepacia* strains have been reported as facultative endophytes of lupine plants or were found to colonise roots of various maize cultivars (Hebbar *et al.,* 1992a; Hebbar *et al.,* 1992b).

Wild type strain *Burkholderia cepacia* L.S.2.4 and its nickel resistant derivative L.S.2.4::*ncc-nre* (Taghavi *et al.,* 2001) were inoculated in perlite and the sterile *Lupinus* seedlings were grown on this substrate for 21 days under controlled environmental conditions. Non-inoculated sterile plants were used as controls. In the absence of NiCl₂, no difference in growth response was observed between the 21 days-old non- inoculated control plants and the inoculated lupine plants when the shoot biomass and length were considered. The roots seemed to be slightly but significantly affected in their growth when *B. cepacia* was added as a wild type strain or as the nickel resistant derivative, indicating that the presence of B. *cepacia* L.S.2.4 has a minor, but positive effect on the root development in the absence of nickel. The nickel concentration in both roots and shoots was measured with A.A.S but was below the detection limit (<2.5 mg/kg DW).

Addition of 0.25 mM NiCl₂ to the perlite resulted in a decrease of the growth parameters when compared to the treatment without NiCl₂, suggesting a toxic effect of the nickel cations. The presence of *B. cepacia*, both wild type and its nickel resistant derivative, did not influence the growth of the plants. No significant differences were observed when root and shoot biomass and length were measured. However, a different response in nickel accumulation was observed when the nickel concentration in the roots was compared for the different treatments (**Figure 1**). A significantly higher total nickel concentration was measured in the lupine roots inoculated with the nickel resistant *B. cepacia* L.S.2.4::*ncc-nre*, while the non-inoculated control plants and the plants inoculated with the wild type strain L.S.2.4 had similar but lower nickel contents. In contrast to the roots, the nickel concentration in the shoots was comparable for the different treatments **(Figure 1).** This is explained by the preferential colonization by *B. cepacia* L.S.2.4 of the *Lupinus* roots. Another reason might be that the nickel ions are complexed by *B. cepacia* L.S.2.4 in the roots, and consequently their transfer to the shoots is blocked.

### Example 5: Decreased phyto-volatilisation of toluene and TCE

Many degradation pathways for organic xenobiotics are located on mobile genetic elements, including plasmids and transposons. Therefore it should be feasible to use natural gene transfer, either based on bi- or tri-parental conjugation or exogenous plasmid isolation (Szpirer *et al.,* 1999), for constructing strains of endophytic bacteria with improved degradation capacity of organic xenobiotics. An example is the transfer the TOM_{31c} plasmid, enabling growth on phenol and toluene, from *B. cepacia* G4 (Shields et al., 1995) into the Ni-resistance strain *B. cepacia* L.S.2.4::ncc-nre. Transconjugants should be selected on their ability to grow on toluene in the presence of 2 mM Ni. In addition the transconjugants will be able to degrade TCE (tri-chloro-ethylene) without induction of the *tom*A gene by toluene, due to the constitutive expression of the toluene-*ortho*-monooxygenase (Shields and Reagin, 1992).

In order to reduce phyto-volatilisation of organic xenobiotics through the plants' stoma, it is suggested to inoculate plants with endophytic bacteria. These bacteria should preferentially colonise the xylem, and should be able to degrade the organic contaminant of interest, such as those mentioned in Table 1. Either natural endophytic bacteria, able to degrade the organic contaminant of interest, or endophytic bacteria equipped either by natural gene transfer or recombinant DNA techniques, can be selected for this purpose.

In case the internal plant concentration of the organic xenobiotic will be too low to efficiently induce the degradation pathway or when the contaminant is degraded due to co-metabolism, mutated pathways that show constitutive expression of degradation should be preferentially introduced in the endophytic bacteria. An example is constitutively expressed toluene-*ortho*-monooxygenase of *B. cepacia* G4 (Shields and Reagin, 1992), which results in constitutive TCE degradation without the need to induce TCE cometabolism by the presence of phenol or toluene.

For phytoremediation of contaminated groundwater, preferentially deep-rooting trees are used, such as poplar or willow. In addition to their large water consumption, trees have the advantage that the retention time of the contaminant in the xylem is quite long (up to two days) allowing sufficient time for in planta degradation of the contaminant by the endophytic bacteria.

Improved phytoremediation of toluene and TCE, resulting in decreased phyto-volatilization, would involve the following steps:
- Selection of the plant species of interest, well adapted to the local climate and geohydrological constraints;
- Isolation of endophytic bacteria from the selected plant species;
- Selection or construction of toluene degrading strains or construction of recombinant stains that constitutively express the degradation pathway, including a toluene-*ortho*-monooxygenase that allows efficient degradation of TCE without the need of cometabolism.
- Inoculation of the selected plants with the endophytic bacteria that are now equipped with the necessary degradation pathways.
- The overall outcome will be improved phytoremediation of toluene and TCE due to increased *in planta* degradation and reduced phyto-volatilisation.
- A similar concept is feasible for any organic xenobiotic that is taken up by plants and for which microbial degradation pathways are available and can be expressed in plant associated endophytic bacteria.

### Employment of reinoculated and not reinoculated plants in experiments with toluene

Reinoculated and not reinoculated plants of Lupinus luteus were grown for a period of three weeks in sterile plastic jars, filed with sterile perlite saturated with ½ concentrated sterile Hoagland's solution. Plants were grown in climate chamber, with constant temperature 22°C; relative humidity 65% and light and dark cycles of 12/12 (described earlier).

After three weeks plants were carefully taken out from jars, all perlit was removed and plant roots were vigorously rinsed in sterile water. Rinsing of the roots in sterile water was done in order to prevent degradation of toluene by bacteria outside the plant. From one reinoculated plant and from one control plant roots and shoots were separated, sterilized for 5 minutes in solution containing 1% active chloride, rinsed three times in sterile water and mixed in 10ml MgSO4 ⁻². 100 µl of the supernatant was plated on three different media (284+gluc; 284+1mM Ni+100Km+gluc; 284+1mM Ni+50Km+toluene).

Subsequently, two reinoculated and one non-reinoculated plant were settled in three separated glass grow chambers. The growchambers have dimensions 29 cm height and diameter 9 cm. Compartment above and compartment under are separated with a glass plate, which have insertion breadth as tree of Lupinus luteus. In each chamber one plant was placed, insertions were closed with gyps so that shoots were in the upper compartment and roots in the lower compartment. Those two compartments were completely separated with no gas exchange between them. The lower compartment was filed with 300 milliliters of sterile, ½ concentrated Hoagland's solution. Each compartment was connected with air source with inflow of 1 liter per hour. Furthermore, each compartment was fitted with a two-linked Tenax traps. Traps were used to capture any transpired or volatilized toluene and were changed at 1 hour to 24 hours intervals. Toluene was added on the beginning of the experiment in the Hoagland's solution in certain concentration.

### Experiment number 1

Toluene was added in concentration of 1000mg/liter.

The experiment was running for 5 days. Columns with Tenax were changed every 24 hours.

Biomass of the plants was measured at the beginning and on the end of the experiment. Results are presented in table 2.2:

**Table 2.2:**

| Biomass of the reinoculated and control plants before and after experiment where toluene was added in concentration 1000mg/l | | | | |
|---|---|---|---|---|
| Growth chamber number | | biomass day 1 | biomass day 5 | Difference |
| 1 | Reinoculated plant | 7.26 g | 9.26 g | 2 g |
| 2 | Reinoculated plant | 7.15 g | 9.17 g | 2.02 g |
| 3 | Control plant | 7.62 g | 8.42 g | 0.8 g |

Biomass of reinoculated plants increased in five days for 2 grams, while biomass of control plant increased 0.8 grams.

In order to determine success of the reinoculation, the bacteria were isolated from reinoculated and not reinoculated plants not used in the experiment. Once the experiment was finished, the bacteria were also isolated from the plants used in the experiment. The results are presented in table 2.3 and 2.4.

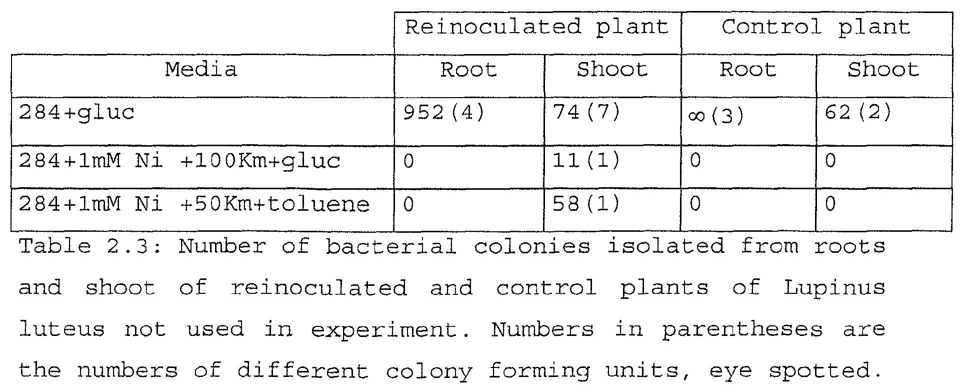

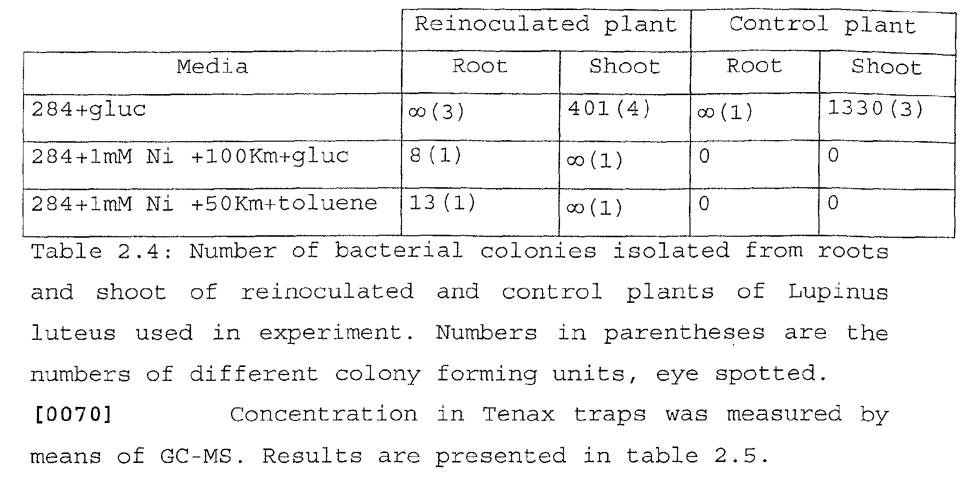

Concentration in Tenax traps was measured by means of GC-MS. Results are presented in table 2.5.

**Table 2.5:**

| Amount of toluene in µg detected in Tenax traps, means by GC-MS | | | | | |
|---|---|---|---|---|---|
| | | Compartment above | | Compartment under | |
| | Measurement in hours | First trap | Control trap | First trap | Control trap |
| Reinoculated plant 1 | 24 | 11.4 | | 154.5 | |
| | 48 | 9.3 | | 126 | |
| | 72 | 0.9 | | 89.4 | |
| | 96 | 78.1 | | 82.6 | |
| | 120 | 5.2 | 14.6 | 104.5 | 110.7 |
| | | 104.9 | 14.6 | 557 | 110.7 |
| | | 119.5 | | 667.7 | |
| | | 787.2 | | | |
| Reinoculated plant 2 | 24 | 37.2 | | 269.6 | |
| | 48 | 1.3 | | 121.7 | |
| | 72 | 1.3 | | 97 | |
| | 96 | 15.5 | | 68 | |
| | 120 | 8.5 | 4.5 | 82.5 | 129.8 |
| | | 63.8 | 4.5 | 638.8 | 129.8 |
| | | 68.3 | | 768.6 | |
| | | 836.9 | | | |
| Control plant | 24 | 0.2 | | 1.4 | |
| | 48 | 0.3 | | 68.1 | |
| | 72 | 42.9 | | 75.1 | |
| | 96 | | | | |
| | 120 | 0.2 | 5.4 | 10.4 | 24.7 |
| | | 43.6 | 5.4 | 155 | 24.7 |
| | | 49 | | 179.7 | |
| | | 228.7 | | | |

### Experiment number 2

Toluene was added in concentration of 500mg/liter.

The experiment was running 3 days. Columns with Tenax were changed every 24 hours.

Biomass of the plants was measured at the beginning and at the end of the experiment. Results are presented in table 2.6:

**Table 2.6:**

| Biomass of the reinoculated and control plants before and after experiment where toluene was added in concentration 500mg/l | | | | |
|---|---|---|---|---|
| Growth chamber | | Biomass day 1 | Biomass day 3 | Difference |
| 1 | Reinoculated plant | 6.99 g | 8.6 g | 1.66 |
| 2 | Reinoculated plant | 7.15 g | 9.01 g | 1.86 |
| 3 | Control plant | 7.44 g | 8.56 g | 0.92 |

Biomass of reinoculated plants increased in three days by nearly 2 grams, while biomass of control plant increased 1.12 grams.

Bacteria were isolated from reinoculated and not reinoculated plants not used in experiment as well as from the plants used in experiment after experiment was finished. Results are presented in table 2.7 and 2.8.

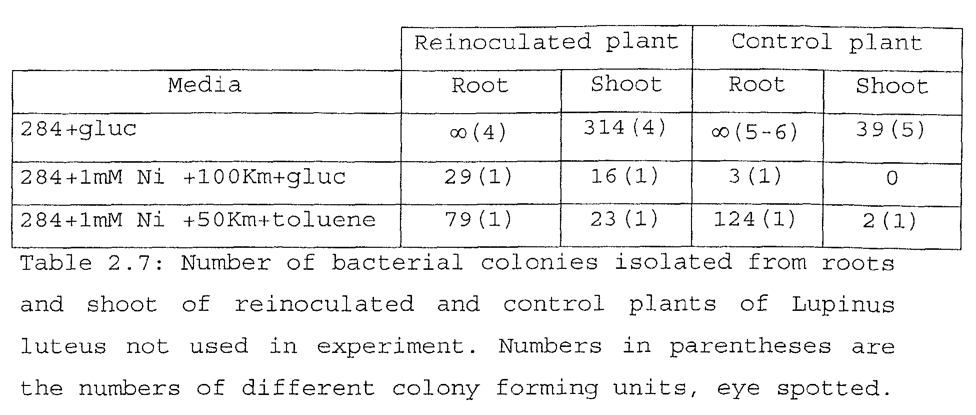

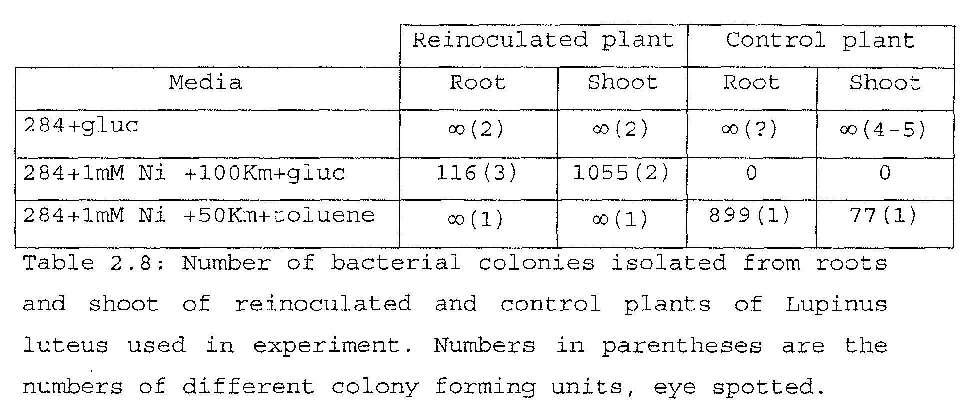

Toluene concentration in Tenax traps was measured by means of GC-MS . Results are presented in table 2.9.

**Table 2.9:**

| Amount of toluene in µg detected in Tenax traps, means by GC-MS | | | | | |
|---|---|---|---|---|---|
| | | Compartment above | | Compartment under | |
| | Measurement in hours | First trap | Control trap | First trap | Control trap |
| Reinoculated plant 1 | 24 | 27.3 | 91.6 | 106.2 | 307.7 |
| | 48 | 22.1 | 6.7 | 128.7 | 134.5 |
| | 72 | 11.9 | 1.6 | 121.2 | 141.1 |
| | | 61.3 | 99.9 | 356.1 | 583.3 |
| | | 161.2 | | 939.4 | |
| | | 1100.6 | | | |
| Reinoculated plant 2 | 24 | 31.6 | 115.4 | 138.6 | 346.0 |
| | 48 | 13.1 | 3.2 | 154.3 | 219 |
| | 72 | 3.4 | 0.2 | 129.8 | 173.5 |
| | | 48.1 | 118.8 | 422.7 | 738.5 |
| | | 166.9 | | 1161.2 | |
| | | 1328.1 | | | |
| Control plant | 24 | 4.2 | 0.7 | 142.9 | 285.2 |
| | 48 | 1.7 | 0.1 | 125.8 | 195.9 |
| | 72 | 0.3 | 0 | 98.1 | 144 |
| | | 6.2 | 0.8 | 366.8 | 625.1 |
| | | 7 | | 991 | |
| | | 998 | | | |

### Toxicity test: influence of different concentrates of toluene on growth of Lupinus luteus

During three weeks, 21 day old Lupinus luteus plants were grown hydroponicly in the presence of a different concentration of toluene. The system was open and therefore nutrient solution was changed the toluene was added every day. The concentration of toluene added to nutrient solution was 0, 100, 200, 300, 400, 500, 600, 700, 800, 900, 1000, 2000 and 3000 milligrams per liter. The biomass of the plant was measured at the day 0, 7, 14 and 21. Results are presented in the graphic 1.

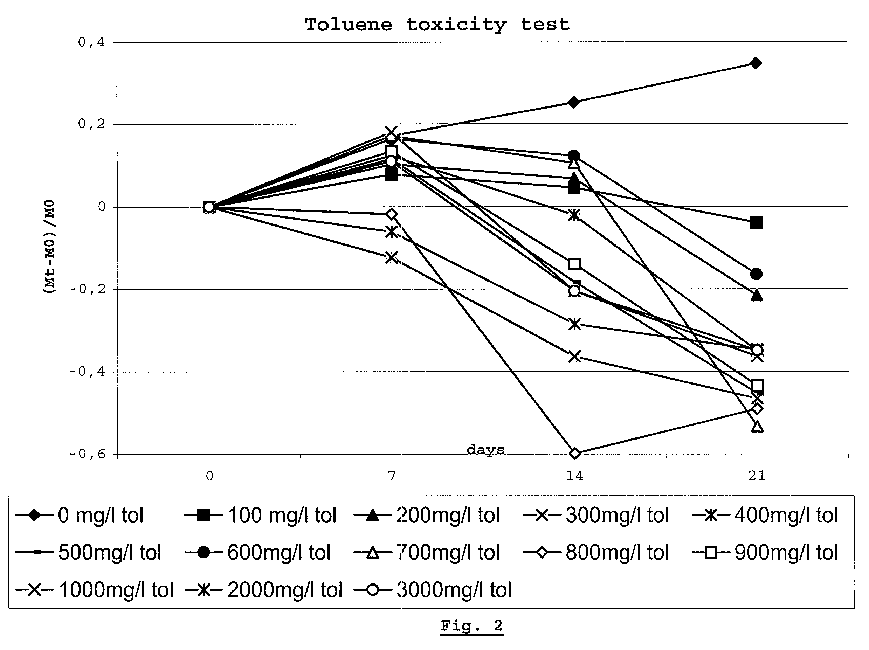

Figure 2 shows the influence of different concentration of toluene on growth of Lupinus luteus . Mt biomass of the plant on the day 7, 14 or 21. MO biomass of the plant on the 0.

This experiment has shown that the exposure of the plant to the lowest, as well as to the highest concentration of the toluene has little influence on the grow of the plants during the first week of experiment. During the second week of the experiment, plants exposed to the highest concentration of toluene had significant retention of growth, what is expressed in the lost of biomass. At the end of the third week, all plants were death except the control plant and plant exposed to toluene at concentration of 100mg per liter nutrient solution.

### Example 6: Decreased accumulation of tri-chloro-acetate (TCA) as metabolite of TCE degradation

Certain organic xenobiotics are partially degraded by plants. However, their efficient remediation is hindered by the accumulation of toxic degradation product. An example is the degradation of TCE by several wild plants (like oak, castor bean and others), which results in the accumulation of phytotoxic TCA (tri-chloro-acetate). The *in planta* accumulation of TCA and consequently its phytotoxicity might be reduced by inoculation with endophytic bacteria able to degrade TCA. Improved phytoremediation of TCE due to reduced accumulation of phytotoxic TCA would involve the following steps:
- Selection of the plant species of interest, well adapted to the local climate and geohydrological constraints;
- Isolation of endophytic bacteria from the selected plant species;
- Selection or construction of TCA degrading strains or construction of recombinant stains that constitutively express the TCA degradation pathway.
- Inoculation of the selected plants with the endophytic bacteria that are now equipped with the necessary TCA degradation pathway.
- The overall outcome will be improved phytoremediation of TCE due to reduced accumulation of phytotoxic TCA and hence survival of trees on higher-contaminated soils or groundwater.
- A similar concept is feasible for any organic xenobiotic that is taken up by plants and for which partial degradation products, microbial degradation pathways are available and can be expressed in plant associated endophytic bacteria.

### Example 7: Decreased toxicity of organic xenobiotics, including agrochemicals such as herbicides

Many herbicides, e.g. of the triazin type or 2,4-dichlorophenoxyacetic acid (2,4-D), are xylem mobile, which means they are taken up by roots and are transported through the xylem to the leaves, where they enfold their action (e.g., inhibition of photosynthesis). 2,4-D functions as a systemic herbicide that can be used to control a range of broad leaf weeds. It is normally applied as a foliar spray and although its exact mode of action is unknown it is transported through the xylem and phloem and through the roots.

Plant-specific endophytic degrader bacteria might be introduced to agricultural crop plants, which are not resistant to the herbicidal action. Endophytical degradation of compounds might therefore be used to make plants selectively resistant against broad-spectrum herbicides.

Bacterial pathways are available for the metabolism of specific organic herbicides e.g. 2,4-dichlorophenoxyacetic acid (2,4-D). Naturally occurring plasmids have been identified that encode genes for the biodegradation and detoxification of 2,4-D (Don and Pemberton, 1981).

*Pseudomonas* sp. strain ADP initiates catabolism of atrazine (a triazin type herbicide) via three enzymatic steps, encoded by *atz*A, -B, and -C, which yield cyanuric acid, a valuable nitrogen source for many bacteria and plants. Plasmid transfer studies indicated that the *atzA,* -B, and -C genes are localised on a 96-kb broad host-range, self-transmissible plasmid, pADP-1, in *Pseudomonas* sp. strain ADP (de Souza *et al.,* 1998).

The construction and use of endophytic bacteria that can biodegrade herbicides and their inoculation on plants may confer on the plant host the ability to resist and detoxify the herbicide. This may provide many benefits including an alternative approach to generating herbicide resistant plants, allow the use of specific herbicides to promote the establishment of phytoremediation plants for in situ bioremediation, and allow the application of existing or future herbicides to a broader range of agricultural plants.

Improved phytoremdiation by the use of herbicide degrading endophytic bacteria would involve the following steps:
- Isolation of endophytic bacteria from the selected plant species.
- Selection or construction of specific herbicide degrading endophytic strains by natural plasmid transfer, e.g. transfer of pJMP5 from *Ralstonia eutropha* JMP365 (Don and Pemberton, 1985) to obtain 2,4-D degradation, or pADP-1 from *Pseudomonas* sp. strain ADP (de Souza *et al.,* 1998) to obtain atrazine degradation.
- Inoculation of the selected plants with the endophytic bacteria that are now equipped with the necessary degradation pathway, e. g. for 2,4-D or atrazine.
- The overall outcome will be improved phytoremediation systems with herbicide degradation/resistance properties, e.g. for 2,4-D or atrazine. Another expected outcome is the selectivity of inoculated agricultural plants to broad-spectrum herbicides, e.g. glyphosate, bromacil and others.

A deposit of the strain Burkholderia cepacia L.S.2.4::ncc-nre has been made according to the Budapest Treaty under the deposit number LMG P-20359 at the BCCM/LMG, Laboratorium voor Microbiologie - Bacterienverzameling, Universiteit Gent, K.L. Ledeganckstraat 35, B-9000 Gent, Belgium on May 3, 2001.

### REFERENCES

1. Arazi, T. et al. The Plant Journal 20: 171-182 (1999).
2. Brewer, E.P. et al. Theo. Appl. Genet. 99: 761-771 (1999).
3. Burd, G.I. Appl. and Environ. Microbiol. 64: 3663-3668 (1998).
4. Cunningham, S.D. et al. In Vitro Cell. Dev. Biol. 29: 207-212 (1993) .
5. Cunningham, S.D. et al. Plant Physiol. 110: 715-719 (1996).
6. De Souza, M.L. et al. Appl. Environ. Microbiol. 64: 2323-2326 (1998).
7. Diels, L. et al. J.Ind.Microbiol. 14:142-153 (1995).
8. Don, R.H. and Pemberton, J.M. J. Bacteriol.145: 681-686 (1981).
9. Don, R.H. and Pemberton, J.M. J. Bacteriol.466-468 (1985).
10. Di Fiore, S. and Del Gallo, M. Azospirillum VI and related microorganisms Springer Verlag, Berlin, Heidelberg (1995).
11. Hebbar, K.P. et al. Soil Biol.Biochem. 24: 989-997 (1992a).
12. Hebbar, K.P. et al. Soil Biol. Biochem. 24: 999-1007 (1992b).
13. Kloepper, J.W. et al. Can. J. Microbiology 38, 1219-1232 (1992).
14. Kloepper, J.W. et al. Phytopathol. 82: 726-727 (1992).
15. Mc Crady J. et al. J. Experimental Botany 38: 1875-1890 (1987).
16. Mergeay, M. et al. J. Bacteriol 162: 328-334 (1985).
17. Mergeay, M. 1997. Microbial resources for bioremediation of sites polluted by heavy metals. J. Wild *et al.*(*eds*) Perspectives in Bioremediation, 65-73.
   Kluwer Academic Publishers.
18. Pfleeger, T. et al. A short-term bioassay for whole plant toxicity. In: Plants for Toxicity Assessment: Second Volume, ASTM STP 1115. J.W. Gorsuch, R.W. Lower, W. Wang and M.A. Lewis (Eds.), American Society for
Testing and Materials, Philadelphia, pp. 355-364 (1991).
19. Salt, D.E. et al. Int. J. Phytorem. 1: 67-79 (1999).
20. Shields, M.S. and Reagin, M. J. Appl. Environ. Microbiol. 58: 3977-3983 (1992).
21. Shields MS Appl. Environ. Microbiol. 61: 1352-1356 (1995).
22. Szpirer, C. et al. Microbiol. 145: 3321-3329 (1999).
23. Taghavi, S. Res. Microbiol. 148: 536-551 (1997).
24. Taghavi, S. et al. Appl and Environ Microbiol 67: 1015-1019 (2001).
25. Thompson P.L. et al. Environ. Toxicol. Chem. 17: 902-906 (1998).
26. Trapp, S. Environmental Toxicology and Chemistry 13 (3): 413-422 (1994).
27. Trapp, S. Pest Management Science (formerly Pesticide Science) 56: 767-778 (2000).
28. Trapp S. Environ. Sci. Technol., in print (2001).

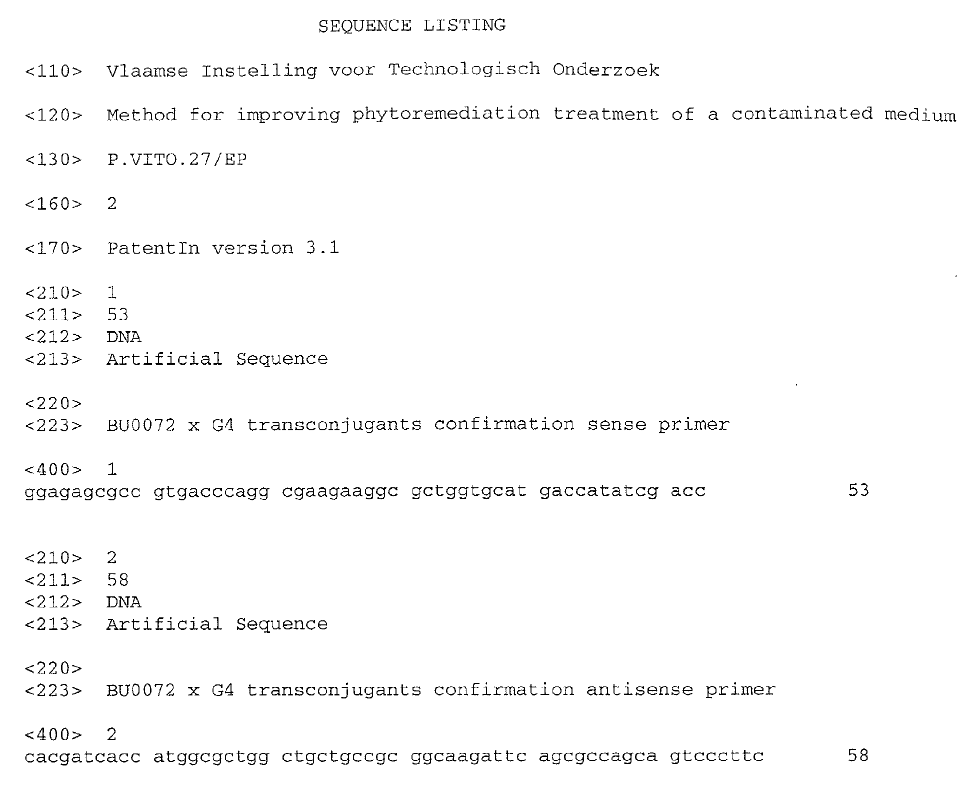

## Claims

1. A method for the phytoremediation treatment of a contaminated medium with at least one element selected from the group consisting of organic pollutants, heavy metals, radionuclides or a mixture thereof, comprising the step of cultivating upon said contaminated medium a plant associated with an endophytic microorganism able to improve the phytoremediation by said plant and the step of recovering the elements present in said plant.

2. The method according to the claim 1, wherein the contaminated medium is a contaminated soil or a contaminated aqueous medium.

3. The method according to the claim 1 or 2, wherein the endophytic microorganism is an endophytic bacteria.

4. The method according to any of the preceding claims 1 to 3, wherein the endophytic microorganism is a genetically modified endophytic microorganism.

5. The method according to any of the preceding claims 1 to 4, wherein the organic pollutant is a water soluble and/or volatile.

6. The method according to any of the preceding claims 1 to 5, wherein the organic pollutant is an agrochemical.

7. The method according to the claim 6, wherein the agrochemical is a herbicide.

8. The method according to any of the preceding claims 1 to 5, wherein the organic pollutant is selected from the group consisting of phenol, chlorophenol, TNT, toluene, benzene, amido-dinitrotoluene, MTBE, BTEX, chlorinated ethenes, organotin compounds, PCBs, PBBs, brominated flame retardants, fluorinated alkylsulfonates, in particular perfluoro-octanyl sulfonate (PFOS).

9. The method according to any of the preceding claims 1 to 6, wherein the heavy metal is selected from the group consisting of the following metals zinc, cadmium, cobalt, nickel, copper, lead, mercury, thallium, barium, boric, selenium, chrome, cesium, strontium, uranium, plutonium, lanthanides or their salt.

10. The method according to any of the preceding claims wherein the endophytic microorganism is present in the vascular system and/or the roots of the plant.

11. The method according to any of the preceding claims wherein the contaminated element is accumulated in the roots, the stem or the leaf of the plant.

12. Plant comprising in its vascular and/or root system a genetically modified endophytic microorganism able to express proteins allowing the degradation or the accumulation of at least one element selected from the group consisting of organic pollutants, heavy metals, radionuclides or a mixture thereof by said plant.
